# EUROPÄISCHE PATENTSCHRIFT

(11) **EP 0 499 837 B1**
(45) Veröffentlichungstag und Bekanntmachung des Hinweises auf die Patenterteilung: **15.05.1996**
(21) Anmeldenummer: 92101324.9
(22) Anmeldetag: 28.01.1992
(51) Int. Cl.: G01N 33/543, G01N 33/531

(54) **Definierte Beschichtung mit rekombinanten Fusionsproteinen aus konstantem Fusionspartner und variablem Antigenanteil in diagnostischen Testsystemen**
A defined coating of fusion proteins from a constant fusion partner and a variable antigenic part in diagnostic test systems
Une couche de protéines de fusion déterminée faite d'un partenaire de fusion constante et une partie antigénique variable dans les systèmes d'essai diagnostiques

(30) Priorität: 21.02.1991 DE 4105400
(43) Veröffentlichungstag der Anmeldung: 26.08.1992
(73) Patentinhaber: BEHRINGWERKE Aktiengesellschaft, 35001 Marburg (DE)
(72) Erfinder: Wieczorek, Leszek, W-3550 Marburg 21 (DE); Harthus, Hans-Peter, W-3550 Marburg (DE); Krupka, Udo, W-3550 Marburg (DE)

(56) Entgegenhaltungen:
- EP-A- 0 293 184
- WO-A-88/04692
- DE-A- 3 523 701
- CHEMICAL ABSTRACTS, vol. 114, no. 19, 13. Mai 1991, Columbus, OH (US); S. KEISSIG et al., Nr. 181719g/
- CHEMICAL ABSTRACTS, vol. 113, no. 25, 17. Dezember 1990, Columbus, OH (US); G. GEORGIOU et al., Nr. 229676z/

## Beschreibung

Die Erfindung betrifft feste Trägermaterialien, z.B. Mikrotitrationsplatten, Kugeln oder Mikrokugeln, Magnetpartikel, Folien, Filterpapiere und ähnliches, die mit Antikörpern gegen rekombinante Fusionsproteine beschichtet sind sowie Verfahren zur reproduzierbaren und definierten Beschichtung dieser Oberflächen mit solchen rekombinanten Fusionsproteinen. Die rekombinanten Fusionsproteine enthalten einen konstanten Fusionspartner und einen variablen Antigenteil, der die Spezifität des Testsystems ausmacht. Die vorgenannten Antikörper sind gegen den konstanten Fusionspartner also den Teil jedes Fusionsproteins gerichtet, der für sich nicht von analytischem Interesse und einheitlich ist.

EP-A-0 293 184 beschreibt ein immunodiagnostisches Gerät worin ein Antikörper und Antigene an einen festen Träger gebunden sind.

Bei der Beschichtung von Oberflächen mit Antigen z.B. Proteinen, die in diagnostischen Testsystemen z.B. ELISA, Latex-Agglutinationstesten usw., eingesetzt werden, ist die chemische Affinität des Antigens ausschlaggebend für die homogene Beschichtung einer Oberfläche, besonders dann, wenn gereinigte Antigene vorliegen. Die Homogenität der Beschichtung ist unter anderem abhängig von den chemischen Eigenschaften des Antigens, wie Hydrophobizität oder Polarität, die wiederum von physiko-chemischen Parametern abhängen wie pH-Wert, Ionenstärke, Molekulargewicht, Verteilungskoeffizienten oder Löslichkeitsverhalten.

Sind in einem Beschichtungssystem zudem noch mehrere Komponenten involviert, erhöht sich die Komplexizität des Reaktionsmechanismus durch Kompetition um die vorhandenen Bindungstellen. Die Kompetition ist wiederum abhängig von den verschiedenen chemischen Eigenschaften der Beschichtungsantigene und kann zum Teil durch die Wahl der Beschichtungskonzentrationen, der Beschichtungskonditionen wie pH-Wert und Ionenkonzentration bzw. -komposition des Beschichtungspuffers oder auch durch sequentielle Beschichtung kontrolliert werden. Allerdings ist dabei immer mit einem erheblichen Verlust einzelner Systemkomponenten zu rechnen, da bei gleichzeitiger Beschichtung von schwach und stark reagierenden Komponenten die Kompetition nur durch Konzentrationserhöhung der schwächer reagierenden zu deren Gunsten verschoben werden kann. Da die unterschiedlichen Verteilungskoeffizienten der Proteine ihre Bindungseigenschaften bestimmen, können auch ihre Gemische durch den Nernst'schen Verteilungssatz beschrieben werden.

Bisher wurde das Problem der Kompetition zwischen mehreren rekombinanten Antigenen bei der Beschichtung von Oberflächen, die in diagnostischen Nachweisverfahren eingesetzt werden, durch eine zeitlich und/oder räumlich getrennte Beschichtungssequenz mit den individuellen rekombinanten Proteinen teilweise vermieden. So wurde bei der Beschichtung von Kugeln mit rekombinanten Antigenen beispielsweise zuerst die Hemisphäre A mit dem Antigen 1 beschichtet und in einem Folgeschritt die Hemisphäre B mit dem Antigen 2. Dieses Prinzip enthält verschiedene verfahrenstechnische Probleme und ist im Fall eines multifaktoriellen Komponentensystems nur mit großen Schwierigkeiten einsetzbar.

Bei der Beschichtung von Mikrotitrationsplatten für den ELISA ist eine sequentielle Beschichtung der Kavitäten mit den individuellen Proteinen derart denkbar, daß zuerst Antigen 1 mit einem Füllvolumen von z.B. 50 µl eingesetzt wird, danach das Antigen 2 mit einem Füllvolumen von 75 µl usw.

Auch dieses Verfahren birgt den Nachteil der zeitlich aufwendigen sequentiellen Beschichtung der Kavitäten in sich, wobei die aufwendigen Wasch- und Beschichtungsschritte besonders fehleranfällig sind. Zudem ist bei dieser Methode nicht die Sicherheit gegeben, daß die Komponenten tatsächlich in der erforderlichen Konzentration an die Festphase gebunden werden.

Der Erfindung liegt die Aufgabe zugrunde, feste Trägermaterialien bereitzustellen, die mit monoklonalen oder polyklonalen Fängerantikörpern mindestens einer Spezifität beschichtet sind, die gegen einen Fusionspartner eines rekombinanten Fusionsproteins gerichtet sind. Durch dieses Trägermaterial wird es möglich, rekombinante Proteine über ihren Fusionspartner in definierter Ausrichtung auf der Oberfläche zu fixieren, wobei es unerheblich ist, inwieweit die rekombinanten Proteinproben noch mit Fremdbestandteilen verunreinigt sind. Der Erfindung liegt ferner die Aufgabe zugrunde, feste Träger bereitzustellen, die zusätzlich beschichtet sind mit einem rekombinanten Antigen oder einem Gemisch aus rekombinanten Proteinen oder Proteinfragmenten, die z.B. für die Diagnose von Infektionskrankheiten von Bedeutung sind, wobei das rekombinante Antigen jeweils an einen zumindest weitgehend konstanten Fusionspartner gekoppelt ist. Durch die Vereinheitlichung über den Einsatz eines für den Fusionspartner monospezifischen Antikörpers ergeben sich folgende Vorteile:
1. Es existiert eine gemeinsame Festphase für verschiedene Spezifitäten
2. Verschiedene rekombinante Antigene können in beliebigen Mischungsverhältnissen ausgebracht werden, so daß auch die Einstellung von Stöchiometrien möglich ist, die den in vivo vorliegenden Verhältnissen entsprechen.
3. Die Beschichtung mit dem rekombinanten Fusionsprotein ist unabhängig von seinen physicochemischen Eigenschaften, sondern allein abhängig von der Antikörper-Antigen-Reaktion.
4. Auch die Mischung von rekombinanten Proteinen mit unterschiedlichen Fusionspartnern ist möglich, sofern die spezifischen Antikörper auf die Träger ausgebracht werden.
5. Die Homogenität der Beschichtung kann durch geeignete Nachweisverfahren kontrolliert werden, z.B. durch Antikörper, die gegen den Beschichtungsantikörper gerichtet sind und daher mit dem festen Träger reagieren. Durch diese Kontrolle kann eine reproduzierbare und qualitativ gleichbleibende Beschichtung gewährleistet werden.
6. Die als Antigene eingesetzten bzw. die Antigene enthaltenden rekombinanten Fusionsproteine müssen nicht notwendigerweise in hochreiner Form präsentiert werden, so daß aufwendige Reinigungsverfahren entfallen können.

Die Lösung der Aufgabe ergibt sich aus den Patentansprüchen 1 bis 7, wie folgt:
1. Festes Trägermaterial, beschichtet mit einem monoklonalen oder polyklonalen Fängerantikörper und mindestens zwei verschiedenen daran gebundenen Fusionsproteinen, von denen jedes einen konstanten Fusionspartner und einen variablen Antigenteil enthält, dadurch gekennzeichnet, daß der Fängerantikörper gegen den konstanten Fusionspartner gerichtet ist und die variablen Antigenteile mindestens zwei verschiedene Antigene von diagnostischem Interesse repräsentieren.
2. Festes Trägermaterial nach Anspruch 1, dadurch gekennzeichnet, daß es aus den Kavitäten einer Mikrotitrationsplatte besteht oder in Form von Kugeln, Mikrokugeln, Filtern, Folien oder Magnetpartikeln vorliegt.
3. Festes Trägermaterial nach Anspruch 2, dadurch gekennzeichnet, daß es eine Mikrotitrationsplatte ist, deren Kavitäten mit 0,05 bis 0,5 µg/cm des Fängerantikörpers pro Kavität beschichtet sind.
4. Festes Trägermaterial nach Anspruch 2, dadurch gekennzeichnet, daß es in Form von Magnetpartikeln vorliegt, die mit 0,005 bis 0,5 µg/cm des Fängerantikörpers beschichtet sind.
5. Verwendung des festen Trägermaterials nach einem der Ansprüche 1 bis 4 zum Nachweis spezifischer Antikörper, die gegen ein Fusionsprotein oder ein Gemisch von Fusionsproteinen gerichtet sind.
6. Verwendung nach Anspruch 5, dadurch gekennzeichnet, daß der Nachweis in Vollblut, Plasma, Serum, Liquor, Sputum oder Urin erfolgt.
7. Verfahren zur Beschichtung eines festen Trägermaterials mit mindestens zwei verschiedenen Antigenen von diagnostischem Interesse, indem
   a) ein monoklonaler oder polyklonaler Fängerantikörper kovalent oder adsorptiv auf dem Trägermaterial fixiert wird und
   b) mindestens zwei verschiedene Fusionsproteine an den Fängerantikörper über eine Antigen-Antikörperbindung gebunden werden,
   dadurch gekennzeichnet, daß der Fängerantikörper an einen konstanten Teil des Fusionsproteins, den konstanten Fusionspartner bindet, so daß an einen variablen Antigenteil des Fusionsproteins eine Substanz von diagnostischem Interesse gebunden werden kann.

Geeignete Fusionsproteine sind Proteine, die aus einem gemeinsamen Segment, dem Fusionspartner und einem durch rekombinante DNA-Techniken daran fusionierten Protein oder Proteinfragment bestehen, wobei unerheblich ist, ob die Fusion N- oder C-terminal ist. Bei einer großen Zahl von anzufusionierenden Proteinen oder Proteinfragmenten mag es zweckmäßig sein, mehr als einen einzigen Fusionspartner zu wählen, in der Regel ist der Fusionspartner jedoch konstant. Bei mehreren verschiedenen Fusionspartnern muß die Festphase mit Antikörpern der entsprechenden Spezifitäten beschichtet werden. Die genannten Proteine oder Proteinfragmente sind diagnostisch oder therapeutisch relevante Proteine, die im ersten Fall für den Nachweis von viral, bakteriell oder parasitär bedingten Erkrankungen herangezogen werden, im zweiten Fall z.B. für die Dosierung und Überwachung humoral applizierter Therapeutika eingesetzt werden können. Bei Infektionskrankheiten können einzelne Proteine zu einer verläßlichen Infektionsdiagnose führen (z.B. Hepatitis B-HBₛAg, HIV 1 - p 24; Cytomegalovirus (CMV) - pp 150-Protein; Treponema pallidum - TMPA - Protein; Toxoplasma gondii - p 61-Protein.) Unerheblich ist hierbei, welcher Herkunft der Fusionspartner dieser Proteine ist, so sind MS2-Polymerase-Fusionen ebenso einsetzbar, wie trp-Fusionen. Bevorzugter Fusionspartner ist die β-Galactosidase aus E. coli, insbesondere ein Segment dieses Proteins mit den Aminosäuren 1 bis 375. Bevorzugte Träger sind Mikrotitrationsplatten und Magnetpartikel, obwohl auch Folien, Kugeln, Mikrokugeln und ähnliches eingesetzt werden können. Der Vorteil dieser Testsysteme liegt in der schnellen Abarbeitbarkeit der eingesetzten Proben. Als bevorzugter Fängerantikörper wird ein monoklonaler Antikörper gegen das N-terminale Segment der β-Galactosidase (Aminosäuren 1 bis 375) eingesetzt, obwohl hier auch polyklonale Antikörper, wenn auch mit geringerem Erfolg eingesetzt werden können, sofern sie eine spezifische Reaktivität gegen den Fusionspartner besitzen.

### Beispiel

### 1. Herstellung der rekombinanten Fusionsproteine

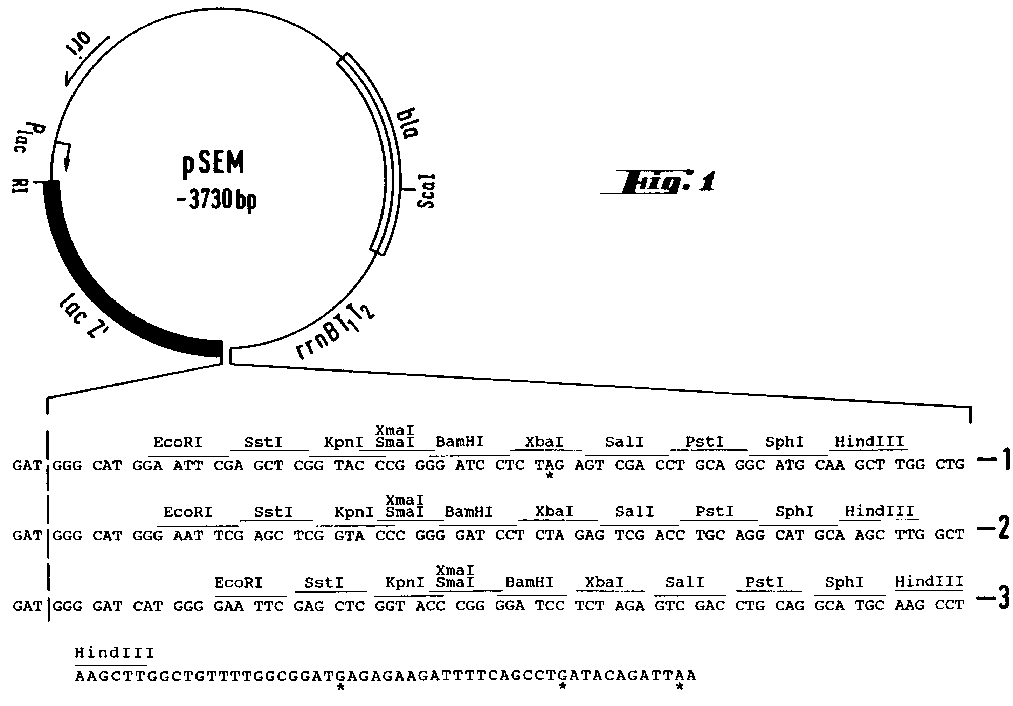

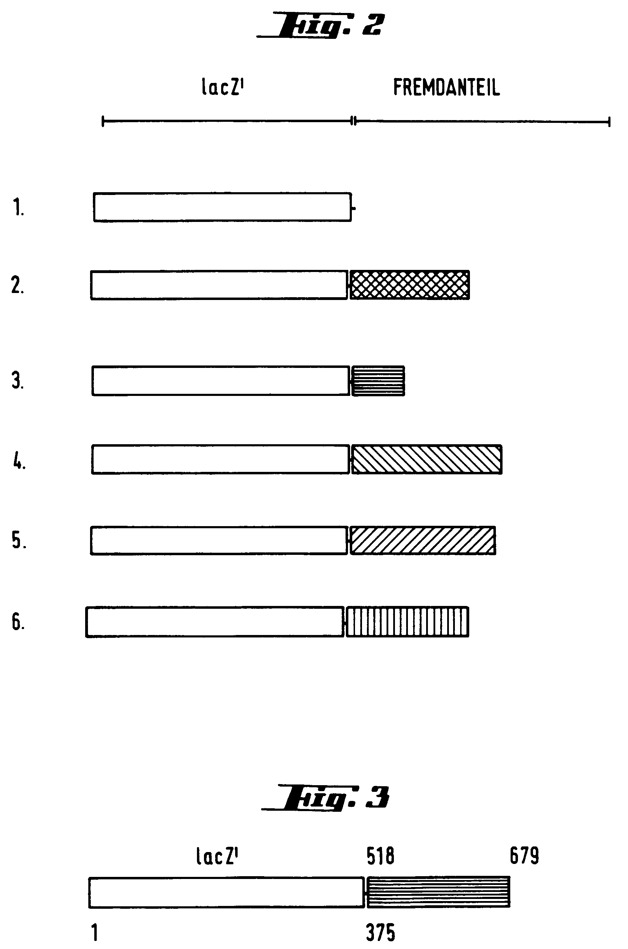

Die hier als Beispiele aufgeführten Fusionsproteine wurden in dem Plasmidvektor-System pSEM exprimiert (Fig. 1; S. Knapp et al., BioTechniques 8 (3), 280 (1990)), das das N-terminale Segment von Aminosäure 1 bis 375 der β-Galactosidase enthält. An die dafür kodierende DNA-Sequenz ist eine "Poly-Linker"-DNA-Sequenz ligiert worden, in die die interessierenden DNA-Fragmente diagnostisch relevanter Genprodukte inseriert werden können. In Fig. 2 sind als Beispiele rekombinante Fusionsproteine dargestellt, die definierte DNA-Segmente aus dem Genom des humanen Cytomegalovirus enthalten und in E. coli exprimiert werden.

### 2. Herstellung der monoklonalen Antikörper gegen die β-Galactosidase von E. coli

Zur Herstellung monoklonaler Antikörper gegen die intakte β-Galaktosidase aus E. coli wurden sechs bis acht Wochen alte Balb/c Mäuse immunisiert. Hierbei wurden pro Maus ca. 10 µg des Enzyms emulgiert in komplettem Freund'schen Adjuvans subcutan und in einem zweiten Fall intraperitoneal injiziert. Jeweils vier bis acht Wochen später folgte eine zweite und dritte Immunisierung. Unmittelbar vor der eigentlichen Fusion wurden die Versuchstiere zusätzlich vier Tage hintereinander intravenös geboostert. Am Tage der Fusion wurden die Milzen steril entnommen und zu Einzelzellen suspendiert. Durch Fusion von 10⁸ Milzzellen mit 2 x 10⁷ Zellen der Myelomzellinie SP 2/0 oder Zellinie X 63 Ag 8653 (Journal of Immunol 173 (1979), 1548 bis 1550) wurden Hybridzellen erzeugt, welche anschließend in einem Selektionsmedium (Dulbecco's Minimal Essentiel Medium, DMEM supplementiert mit 20 % fötalem Kälberserum FKS, 0,1 mmol Hypoxanthin, 0,4 mmol Aminopterin sowie 16 mmol Thymidin) auf Kulturplatten mit 24 Kavitäten (Fa. Costan) in einer Konzentration von 10⁶ Zellen/Kavität ausgesät wurden. Zwei bis drei Wochen später wurden aus den Kavitäten einzelne Zellkolonien isoliert und in jeweils eine Kavität einer neuen Kulturplatte übertragen. Nach weiteren zwei bis drei Tagen wurden die Kulturüberstände mit Hilfe eines Enzymimmunoassays auf die Anwesenheit von β-Galactosidase-spezifischen Antikörpern durchsucht. Die Überstände wurden auf β-Galactosidase-beschichteten Mikrotitrationsplatten inkubiert und gegebenenfalls vorhandene spezifische Antikörper durch ein Anti-Maus/Peroxidase-Immunglobulin Konjugat-Reaktion nachgewiesen. Positive Zellinien wurden als Aszites in IFA(inkomplettes Freund'sches Adjuvans)-vorbehandelten Balb/c-Mäusen propagiert. Die Reinigung erfolgte über Ammoniumsulfatfällung sowie Protein A-Chromatographie. Die Reinheit wurde mittels HPLC sowie Gelelektrophorese überprüft, die Immunglobulinklassen durch Immundiffusion nach Ouchterlony bestimmt.

### 3. Charakterisierung der monoklonalen Antikörper

Zellextrakte der Transformanten und Extrakte des Plasmidfreien, β-Galaktosidase-defizienten E.coli Stammes MC4100 (M. J. Casadaban, J. Mol. Biol. 104, S. 541-555 (1976)) sowie ein Protein-Mix zur Molekulargewichtsbestimmung wurden unter denaturierenden Bedingungen in SDS-Polyacrylamidgelen aufgetrennt und anschließend auf Nitrocellulose-Filterpapier transferiert. Die einzelnen Blots wurden mit den verschiedenen monoklonalen Antikörpern inkubiert und entwickelt. Der monoklonale Antikörper 87-55/60 beispielsweise erkannte in diesem System nur Polypeptide, die Sequenzen aus dem Bereich 1 - 375 der β-Galaktosidase enthalten aber keine Polypeptide aus dem Bereich 376 bis 1021 der β-Galaktosidase und auch keine Polypeptide, die durch den E.coli Stamm MC4100 spezifiziert sind. Aufgrund des Reaktionsmusters mit definierten β-Galaktosidase-Fragmenten konnte so bewiesen werden, daß der monoklonale Antikörper 87-55/60 mit einer antigenen Determinante im aminoterminalen Bereich der β-Galaktosidase von Serin 1 bis Asparaginsäure 375 reagiert.

### 4. Herstellung einer mAK-beschichteten Mikrotitrationsplatte

Ein geeigneter monoklonaler Antikörper, der gegen die N-terminale Region der β-Galactosidase gerichtet ist, wird auf eine Konzentration von 0,1 µg Protein je ml Lösung mit physiologischem Phosphatpuffer (PBS) eingestellt und ein Volumen von 100 µg je Kavität einer Mikrotitrationsplatte ausgebracht. Zur Adsorption wird diese Platte über Nacht bei +4°C, 16 Stunden inkubiert und darauf die Beschichtungslösung durch Absaugen entfernt. Anschließend wird die Oberfläche gewaschen mit einem 50 mM Tris/60 mM EDTA-Puffer (pH 7,2) und anschließend mit einer Lösung von Schutzprotein, z.B. Lactoferrin, humanes Serumalbumin, Rinderserumalbumin, oder auch Detergenzien, z.B. Tween®, Cholsäure und ihre Derivate, Aminoxid u. ä., für 45 Minuten bis 2 Stunden inkubiert, um nichtabgesättigte Bindungsstellen zu maskieren Anschließend wird die Oberfläche zweimal mit PBS gewaschen, getrocknet und kann in dieser Form sofort verwendet oder auch gelagert werden.

### 5. Beschichtung von Mikrotitrationsplatten mit rekombinantem Fusionsprotein enthaltend ein Antigen

Wie unter 4. wird der geeignete monoklonale Mausantikörper, der gegen die N-terminale Region der β-Galaktosidase gerichtet ist, wird in einer Konzentration von 0,1 µg Protein je Kavität in physiologischen Phosphatpuffer (PBS) verdünnt und auf die ELISA-Platte ausgebracht. Nach Adsorption des Antikörpers wird die Beschichtungslösung entfernt, die Oberfläche gewaschen und mit einer 0,1 bis 5 %-igen Lösung von Schutzprotein, z.B. Lactoferrin, humanes Serumalbumin, Rinderserumalbumin, oder auch Detergenzien, z.B. Tween®, Desoxycholat, Aminoxid usw., für 45 min bis 2 hrs inkubiert, um nicht-abgesättigte Bindungsstellen zu maskieren. Anschließend wird die Oberfläche zweimal mit PBS gewaschen, und das Fusionsprotein bzw. -gemisch in geeigneter Verdünnung ausgebracht. Nach zweistündiger Inkubation ist die Reaktion des Fusionsproteins mit dem monoklonalen Antikörper abgeschlossen. Unerheblich ist dabei, um welches Antigen es sich handelt, solange den eingesetzten Fusionsproteinen der für den monoklonalen Antikörper relevante Epitopteil des N-terminalen β-Galaktosidase-Fragments gemeinsam ist. Unterschiedliche physikochemische Eigenschaften der eingesetzten Proteine, z.B. ihre Ladung, Hydrophobizität etc., spielen im Falle von Gemischen rekombinanter Proteine keine Rolle, da die reaktive Komponente nicht vom - Fremdanteil des Hybridproteins beigesteuert wird, sondern von der gemeinsamen β-Galaktosidase-Komponente. Dadurch ist ein einheitliches und in der Bindungskinetik eindeutig festgelegtes Bindungsverhalten vorgegeben, das allein durch den β-Galaktosidaseteil der Hybridproteine determiniert wird. Da die Avidität des monoklonalen Antikörpers zu den individuellen Proteinen gleich groß ist, ist die Bindung des Antigens an den Antikörper als ein rein statistisch bestimmter Vorgang zu bewerten. Im Unterschied zur Beschichtung mit heterologen Komponenten, wie z.B. synthetischen Peptiden aus unterschiedlichen Bereichen eines Proteins oder Homogenaten von Zellen, ist hierbei nicht die Gefahr gegeben, daß einzelne Bestandteile aufgrund ihrer überaus günstigen Bindungseigenschaften überrepräsentiert sind und andere wesentliche Bestandteile durch Kompetition verdrängt worden sind. Tab. 1 dokumentiert anhand von 37 Seren (9 CMV negativ, 28 CMV positiv) die grundsätzliche Durchführbarkeit des Testaufbaus.

### 6. Definierte Beschichtung von Mikrotitrationsplatten mit einem Gemisch aus zwei Antigenen

Eine Mikrotiterplatte wird in einem Volumen von 150 µl mit 0,1 µg monoklonalem anti-β-Galactosidase-Antikörper durch Adsorption über Nacht beschichtet und nach zweimaligem Waschen mit 50 mM Tris-HCl und 50 mM EDTA (pH 7.2) jeweils mit pXP1-Protein (Fig. 2 und Fig. 3) als erstem rekombinanten Fusionsprotein und pSEML3 Protein (Fig. 2) als zweitem rekombinantem Protein beschichtet. Die Beschichtung erfolgt in der Weise, daß insgesamt 50 ng eines Fusionsproteingemisches eingesetzt werden, wobei der prozentuale Anteil des pXP1-Proteins von 100 Prozent in 20-Prozent-Schritten auf 0 Prozent abnimmt, die in gleicher Weise der Anteil des pSEML3-Proteins im Gemisch dagegen zunahm. Nach einer Inkubation bei 37°C für eine Stunde werden die Kavitäten entleert, mit obiger Waschlösung zweimal gespült und mit zwei humanen Seren mit Präferenz zu jeweils einem der oben genannten rekombinanten Proteinen in einer Verdünnung von 1:50 für eine Stunde inkubiert. Anschließend wird die Serumprobe wieder aus der Kavität entfernt und nach zweimaligen Waschen mit oben aufgeführtem Puffer ein geeignetes anti-humanes Immunglobulin-G-Peroxidase Konjugat aufgegeben.

Die Immunreaktion wird anschließend mit einem geeigneten Substrat, z.B. Tetramethylbenzidin, photometrisch nachgewiesen. Im gleichen Umfang, in dem der Anteil des einen rekombinanten Proteins in dem Gemisch abnimmt, nimmt auch das spezifische Signal ab, das durch die Seroreaktivität aufgezeichnet wird. Entsprechend gegenläufig verhält sich das andere Serum. Auf diese Weise läßt sich am Schnittpunkt beider Titrationskurven
a) das optimale Beschichtungsverhältnis der Fusionsproteine bestimmen,
b) das reale Beschichtungsverhalten von Antigenmischungen auf Mikrotiterplatten nachvollziehen,
c) und bei Standardisierung der eingesetzten Seren eine reproduzierbare Beschichtungsqualität gewährleisten.

### 7. Beschichtungskontrolle von ELISA-Mikrotiterplatten

Bei der Beschichtung von Elisa-Platten mit rekombinanten Fusionsproteinen wird die Einstellung der Beschichtungskonzentration über die Proteinkonzentration vorgenommen. Mit einem geeigneten murinen monoklonalen Antikörper gegen β-Galaktosidase kann die aktuelle Antigenkonzentration nach der Beschichtung mit Hilfe einer Eichkurve als Referenz ermittelt werden und damit die Beschichtungskonzentration optimiert werden. Weiterhin können Signalverluste oder ähnliche Qualitätsverluste gelagerter Platten im Rahmen von Qualitätskontrollmaßnahmen detektiert werden und unbrauchbare Produktionschargen ausgemustert werden. Damit ist ebenfalls die Kontrollmöglichkeit zur langfristigen Qualitätsüberwachung angelieferter Mikrotitrationsplatten-Chargen in Bezug auf die Standardisierung der Prüfverfahren möglich. Am Beispiel des rekombinanten CMV-Proteins pXP1 ist dieses Verfahren im folgenden beschrieben: Eine Mikrotitrationsplatte wird mit einer Meßlösung des rekombinanten Proteins, deren genaue Konzentration bekannt ist, in definierten Verdünnungsstufen analog dem in der Produktion angewendeten Verfahren beschichtet. Die Verdünnungsstufen sind so eingestellt, daß sie mindestens je eine Größenordnung unter bzw. über der in der Produktion eingesetzten Konzentration liegen. Die bei der sich anschließenden ELISA-Prozedur gefundenen Werte werden in Bezug gesetzt zu einem gleichzeitg mitgeführten Nullwert (entspricht einer Beschichtungsprozedur mit dem Beschichtungspuffer ohne dem Testantigen) ; zur statistischen Absicherung werden geeigneterweise Mehrfachbestimmungen durchgeführt. Die gefundenen Extinktionswerte der ELISA-Auswertung entsprechen dem Verlauf einer Verdünnungseichkurve, die jetzt als Konzentrationsbezugssystem für unbekannte Probe dienen kann. Eine zu kontrollierende Mikrotitrationsplatte kann nach Entwicklung im ELISA durch Interpolation der gefundenen Werte auf eine korrekte Beschichtungskonzentration hin geprüft werden. Weiterhin kann die Gleichmäßigkeit des Beschichtungsvorgangs, die Qualität der eingesetzten Reagenzien anhand von Maßlösungen und ihre Stabilität und Lagerfähigkeit überprüft werden.

**Tabelle 1**

| Serum-Nr. | Bewertung | CMV | pXP1 |
|---|---|---|---|
| 56642 | - | 28 | 60 |
| 56646 | - | 29 | 40 |
| 56941 | - | 30 | 10 |
| 282904 | - | 34 | 107 |
| 56668 | - | 38 | 61 |
| 286132 | - | 41 | 31 |
| 56947 | - | 48 | 12 |
| 56949 | - | 53 | 23 |
| 81776 | - | 108 | 103 |
| 285544 | + | 279 | 619 |
| 310990 | + | 577 | 557 |
| 240516 | + | 752 | 2016 |
| 282852 | + | 817 | 1225 |
| 286574 | + | 942 | 1476 |
| 286139 | + | 1001 | 909 |
| 311041 | + | 1086 | 2500 |
| 282900 | + | 1099 | 2008 |
| 240511 | + | 1123 | 2500 |
| 56640 | + | 1378 | 2500 |
| 310955 | + | 1382 | 2136 |
| 56643 | + | 1386 | 1173 |
| 286602 | + | 1394 | 1079 |
| 81841 | + | 1405 | 566 |
| 270091 | + | 1467 | 1173 |
| 285557 | + | 1474 | 2393 |
| 270114 | + | 1488 | 587 |
| 56645 | + | 1541 | 1628 |
| 81873 | + | 1575 | 2500 |
| 286174 | + | 1580 | 1991 |
| 270099 | + | 1595 | 1468 |
| 56961 | + | 1730 | 1536 |
| 311031 | + | 1779 | 2004 |
| 56963 | + | 1956 | 1336 |
| 56641 | + | 2148 | 1650 |
| 283754 | + | 2192 | 1740 |
| 600164 | + | 2234 | 2048 |
| 600069 | + | 2500 | 2500 |

### Legende zu Fig. 1

### Expressionsvektor für Fusionsproteine aus Escherichia coli

Die E. coli Plasmid Vektoren pSEM-1, pSEM-2 und pSEM-3 dienen zur Expression von Proteindomänen in Bakterien. Diese Vektoren enthalten eine rudimentäre Form des E. coli lacZ Gens (lacZ'), das nur die N-terminalen Aminosäuren 1 bis 375 der β-Galaktosidase (β-Gal) kodiert. Eine daran anschließende Polylinker-Region mit mehreren Klonierungsstellen in den drei möglichen Leserahmen erlaubt die Konstruktion von Fusionsproteinen auf lacZ'-Basis. Die Transkription des Fusionsgens wird durch die Derepression des lac-Promotors (plac) gesteuert und durch die rrnB-Transkriptionsterminatoren T1 und T2 proximal zur Polylinker-Region gestoppt. Weiterhin sind Translationsstops (*) in allen drei Leserahmen unmittelbar hinter der Polylinker-Region eingefügt worden (S. Knapp, M. Bröker, E. Amann, Biotechniques 8, 280-281 [1990]).

### Legende zu Fig. 2

### Schematischer Aufbau von lacZ'-Fusionsproteinen

Alle besprochenen Fusionsproteine enthalten die Aminosäuren 1 bis 375 der β-Galaktosidase (lacZ'), daran anschließend zusätzliche Aminosäuren, die von der Polylinker-Region kodiert werden sowie optional die jeweiligen klonierten Proteindomänen. Beispielhaft sind hier Konstrukte des humanen Cytomegalovirus (HCMV) mit Fremdanteilen aus unterschiedlichen Gensegmenten aufgeführt:
- 1. pSEM-1/2/3: Grundkonstrukt ohne Fremdanteil;
- 2. pXP1: Fremdanteil Nukleotidsequenz 1632 - 2118 des HCMV-Gens pp150, entspricht 162 Aminosäuren;
- 3. pSEML3: Fremdanteil Nukleotidsequenz 2686 - 2946 des HCMV-Gens pp150, entspricht 87 Aminosäuren;
- 4. pSEML1V2: Fremdanteil Nukleotidsequenz 873 - 1514 des HCMV-Gens pp150, entspricht 213 Aminosäuren;
- 5. pHE68-1: Fremdanteil Nukleotidsequenz 548 - 1136 des HCMV-Gens pp65, entspricht 196 Aminosäuren;
- 6. pPS28: Fremdanteil Nukleotidsequenz 40 - 540 des HCMV-Gens pp28, entspricht 167 Aminosäuren

### Legende zu Fig. 3

### Schematischer Aufbau des pXP1-Proteins

Das rekombinante pXP1-Protein enthält neben den Aminosäuren 1 bis 374 der rudimentären β-Galaktosidase (lacZ'; E. coli) und den von einer Polylinker-Region kodierten Aminosäuren eine Proteindomäne aus dem pp150-Protein des humanen Cytomegalovirus, dessen DNA-Fragment (Basenpaare 1632 - 2118; 162 Aminosäurereste) als C-terminale Genfusion an das Vektorfragment kloniert wurde.

## Patentansprüche

1. Festes Trägermaterial, beschichtet mit einem monoklonalen oder polyklonalen Fängerantikörper und mindestens zwei verschiedenen daran gebundenen Fusionsproteinen, von denen jedes einen konstanten Fusionspartner und einen variablen Antigenteil enthält, dadurch gekennzeichnet, daß der Fängerantikörper gegen den konstanten Fusionspartner gerichtet ist und die variablen Antigenteile mindestens zwei verschiedene Antigene von diagnostischem Interesse repräsentieren.

2. Festes Trägermaterial nach Anspruch 1, dadurch gekennzeichnet, daß es aus den Kavitäten einer Mikrotitrationsplatte besteht oder in Form von Kugeln, Mikrokugeln, Filtern, Folien oder Magnetpartikeln vorliegt.

3. Festes Trägermaterial nach Anspruch 2, dadurch gekennzeichnet, daß es eine Mikrotitrationsplatte ist, deren Kavitäten mit 0,05 bis 0,5 µg/cm des Fängerantikörpers pro Kavität beschichtet sind.

4. Festes Trägermaterial nach Anspruch 2, dadurch gekennzeichnet, daß es in Form von Magnetpartikeln vorliegt, die mit 0,005 bis 0,5 µg/cm des Fängerantikörpers beschichtet sind.

5. Verwendung des festen Trägermaterials nach einem der Ansprüche 1 bis 4 zum Nachweis spezifischer Antikörper, die gegen ein Fusionsprotein oder ein Gemisch von Fusionsproteinen gerichtet sind.

6. Verwendung nach Anspruch 5, dadurch gekennzeichnet, daß der Nachweis in Vollblut, Plasma, Serum, Liquor, Sputum oder Urin erfolgt.

7. Verfahren zur Beschichtung eines festen Trägermaterials mit mindestens zwei verschiedenen Antigenen von diagnostischem Interesse, indem
a) ein monoklonaler oder polyklonaler Fängerantikörper kovalent oder adsorptiv auf dem Trägermaterial fixiert wird und
b) mindestens zwei verschiedene Fusionsproteine an den Fängerantikörper über eine Antigen-Antikörperbindung gebunden werden,
dadurch gekennzeichnet, daß der Fängerantikörper an einen konstanten Teil des Fusionsproteins, den konstanten Fusionspartner bindet, so daß an einen variablen Antigenteil des Fusionsproteins eine Substanz von diagnostischem Interesse gebunden werden kann.

## Claims

1. A solid support material coated with a monoclonal or polyclonal trapping antibody and at least two different fusion proteins linked thereto, each of which contains a constant fusion partner and a variable antigen part, wherein the trapping antibody is directed against the constant fusion partner, and the variable antigen parts represent at least two different antigens of diagnostic interest.

2. A solid support material as claimed in claim 1, which comprises the cavities of a microtiter plate or is in the form of beads, microbeads, filters, films or magnetic particles.

3. A solid support material as claimed in claim 2, which is a microtiter plate whose cavities are coated with 0.05 to 0.5 µg/cm of the trapping antibody per cavity.

4. A solid support material as claimed in claim 2, which is in the form of magnetic particles which are coated with 0.005 to 0.5 µg/cm of the trapping antibody.

5. The use of the solid support material as claimed in any of claims 1 to 4 for detecting specific antibodies which are directed against a fusion protein or a mixture of fusion proteins.

6. The use as claimed in claim 5, wherein the detection takes place in whole blood, plasma, serum, cerebrospinal fluid, sputum or urine.

7. A process for coating a solid support material with at least two different antigens of diagnostic interest by
a) immobilizing a monoclonal or polyclonal trapping antibody covalently or by adsorption on the support material and
b) linking at least two different fusion proteins to the trapping antibody via an antigen-antibody binding,
wherein the trapping antibody binds the constant fusion partner at a constant part of the fusion protein so that a substance of diagnostic interest can be bound to a variable antigen part of the fusion protein.

## Revendications

1. Matériau support solide, recouvert d'un anticorps liant monoclonal ou polyclonal, lié à au moins deux protéines de fusion différentes, faites chacune d'un partenaire de fusion constante et d'une partie antigénique variable, caractérisé en ce que l'anticorps liant est orienté contre le partenaire de fusion constant, et en ce que les parties antigéniques variables représentent au moins deux antigènes différents d'intérêt diagnostique.

2. Matériau support solide selon la revendication 1, caractérisé en ce qu'il constitue les cavités d'une plaque de microtitration ou en ce qu'il se présente sous forme de billes, de microbilles, de filtres ou de particules magnétiques.

3. Matériau support solide selon la revendication 2, caractérisé en ce qu'il est une plaque de microtitration dont les cavités sont recouvertes par 0,05 à 0,5 µg/cm par cavité de l'anticorps liant.

4. Matériau support solide selon la revendication 2, caractérisé en ce qu'il se présente sous forme de particules magnétiques, qui sont recouvertes par 0,05 à 0,5 µg/cm de l'anticorps de piégeage.

5. Utilisation du matériau support solide selon l'une des revendications 1 à 4 pour la détection d'anticorps spécifiques qui sont dirigés contre une protéine de fusion ou un mélange de protéines de fusion.

6. Procédé selon la revendication 5, caractérisé en ce que l'on effectue la détection dans du sang complet, du plasma, du sérum, du liquide céphalo-rachidien, des crachats ou de l'urine.

7. Procédé d'enduction d'un matériau support solide avec au moins deux antigènes différents d'intérêt diagnostique, dans lequel
a) est fixé sur le matériau support un anticorps liant monoclonal ou polyclonal, de manière covalente ou par adsorption et
b) par une liaison antigène-anticorps au moins deux protéines de fusion différentes sont liées à l'anticorps liant,
caractérisé en ce que, l'anticorps liant se lie avec le partenaire de fusion constant par une partie constante de la protéine de fusion, de manière que l'on puisse lier une substance d'intérêt diagnostique à une partie antigénique variable de la protéine de fusion.
